# EUROPEAN PATENT SPECIFICATION

(11) **EP 1 764 629 B1**
(45) Date of publication and mention of the grant of the patent: **02.05.2012**
(21) Application number: 06007260.0
(22) Date of filing: 06.04.2006
(51) Int. Cl.: G01S 15/89, A61B 8/00

(54) **Ultrasound diagnostic system and method for rotating ultrasound image**
Ultraschall Diagnosesystem und Verfahren zur Drehung von Ultraschallbild
Système de diagnostic à ultrason et méthode pour la rotation d'image ultrasonore

(30) Priority: 14.09.2005 KR 20050085562
(43) Date of publication of application: 21.03.2007
(73) Proprietor: SAMSUNG MEDISON CO., LTD., Kangwon-do (KR)
(72) Inventor: Kim, Nam Woong, Seoul 135-280 (KR)
(74) Representative: Lorenz, Werner

(56) References cited:
- US-A- 5 540 229
- US-A- 6 117 078
- US-A1- 2002 097 897
- US-A1- 2002 138 007
- US-B1- 6 203 498

## Description

The present invention generally relates to an ultrasound diagnostic system, and more particularly to an ultrasound diagnostic system and a method for rotating an ultrasound image according to claims 1 and 7..

Generally, an ultrasound diagnostic system is used to obtain an image of soft tissue dislocation or blood flow by irradiating ultrasound signals from a surface of the object to a desired portion and using the information acquired from reflected ultrasound signals (ultrasound echo signals). When compared to other image diagnostic devices such as an X-ray diagnostic device, an X-ray Computerized Tomography ("CT") Scanner, a Magnetic Resonance Image ("MRI") and a nuclear medicine diagnostic device, the ultrasound diagnostic system is relatively small and inexpensive. Further, the ultrasound diagnostic system can display the ultrasound image in real time and is fairly safe since there is no exposure to X-rays. Thus, the ultrasound diagnostic system is widely used for diagnosing a heart, an abdomen, a urinary organ and obstetrics.

Particularly, the ultrasound diagnostic system comprises at least one probe, which has a plurality of one-dimensional ("1D") or two-dimensional ("2D") transducers and properly adjusts the timing of applying input pulses to the transducers in order to transmit the condensed ultrasound beam along a transmission scan line to a target. Then, the ultrasound echo signals reflected from the target are inputted to the transducers at different timing. Each transducer then outputs the ultrasound echo signals to a beam former.

As illustrated above, the conventional ultrasound diagnostic system forms an ultrasound image (preferably a 3D ultrasound image) based on the ultrasound echo signals acquired through the probe. It can also display the formed ultrasound image on a display.

In the conventional ultrasound diagnostic system, however, a user typically handles the probe with one hand while using the other hand to handle the control panel of the ultrasound diagnostic system. After displaying the obtained ultrasound image on a display, the user then handles the track ball and a number of buttons on the control panel in order to rotate the displayed ultrasound image to a certain direction or predetermined angle. Therefore, the conventional ultrasound diagnostic system is disadvantageous since it is difficult to rotate the displayed ultrasound image to the predetermined direction or angle.

US 5,540,229 describes a system and a method according to the preamble of claim 1. In this system there is used a 2D-probe for 2D-echography and a 3D-Probe for 2D-echography.

US 6 203, 498 B 1 discloses a hand held ultrasonic imaging device which is provided as an integral unit with all components and circuitry necessary for both image acquisition and image display included in a single enclosure.

In order to solve the above deficiency, there is provided an ultrasound diagnostic system, and a method for rotating the ultrasound image according to claims 1 and 7.

In accordance with the present invention, it is possible for a user to easily rotate the displayed ultrasound image with one hand.

The above and other objects and features of the present invention will become apparent from the following description of preferred embodiments given in conjunction with the accompanying drawings, in which:
Fig. 1 is a block diagram showing an ultrasound diagnostic system according to the first embodiment of the present invention.
Fig. 2 is a flowchart for describing the operation of an ultrasound diagnostic system according to the first embodiment of the present invention.
Fig. 3 is a block diagram for describing an ultrasound diagnostic system according to the second embodiment of the present invention.
Fig. 4 is a flowchart for describing the operation of an ultrasound diagnostic system according to the second embodiment of the present invention.
Fig. 5 is a block diagram for describing an ultrasound diagnostic system according to the third embodiment of the present invention.
Fig. 6 is a flowchart for describing the operation of an ultrasound diagnostic system according to the third embodiment of the present invention.
Fig. 7 is a flowchart for describing the operation of rotating an ultrasound image by using a 3D position sensor according to the third embodiment of the present invention.
Fig. 8 is a flowchart for describing the operation of rotating an ultrasound image by using a joystick according to the third embodiment of the present invention.

(The First Embodiment)

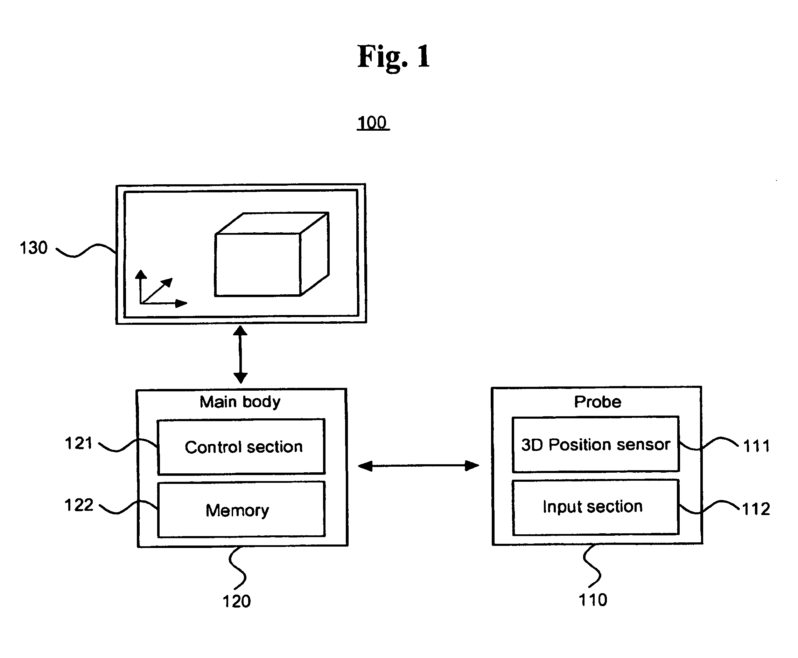

Fig. 1 is a block diagram showing an ultrasound diagnostic system, which is constructed in accordance with the first embodiment of the present invention.

The ultrasound diagnostic system 100, which is shown in Fig. 1, comprises a probe 110, a main body 120 and a display 130. The probe 110 comprises a 3D position sensor 111 and an input section 112. The probe 110 also comprises a plurality of 1D, 2D or 3D transducers (not shown), which can send a condensed ultrasound beam along the transmission scan line to a target (not shown) by properly delaying the timing of applying input pulses. Such transducers further receive ultrasound echo signals reflected from the target at different timing of reception.

The 3D position sensor 111 detects the present position of the probe 110 and generates position information of the probe 110. As for the 3D position sensor 111, any device may be used as long as the device can detect the position of the probe 110. For example, a gyro can be used as the 3D position sensor 111. The input section 112 receives ultrasound image information and reference position setting information of the probe 110.

The main body 120 comprises a control section 121 and a memory 122 for composing an ultrasound image based on the ultrasound echo signals acquired through the probe 110. It is further configured to display the composed ultrasound image on a display 130 and rotate the ultrasound image on the display 130 according to the position change, that is, the rotation of the probe 110. The main body 120 also comprises a beam former, an image signal processor, a scan converter and an image processor (not shown).

The control section 121 controls the overall operation of the ultrasound diagnostic system 100. Particularly, the control section 121 rotates the ultrasound image displayed on the display 130 based on the position information transmitted from the 3D position sensor 111 of the probe 110. The memory 122 stores the reference position information for determining the position change of the probe 110. The reference position information will be described below in detail.

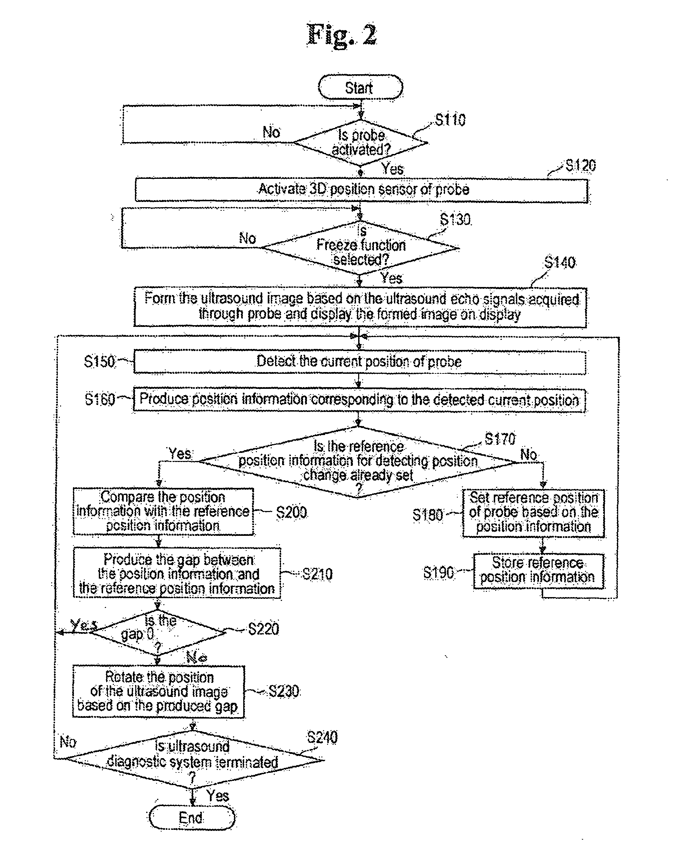

The operation of the ultrasound diagnostic system, which is in accordance with the first embodiment of the present invention, will be described below in view of Figs. 1 and 2. Fig. 2 is a flowchart for describing the operation of the ultrasound diagnostic system, which is in accordance with the first embodiment of the present invention.

As illustrated in Fig. 2, if a user activates the probe 110 by operating the ultrasound diagnostic system 100 (step S110), the control section 121 activates the 3D position sensor 111 of the probe 110 (step S120). If the user then selects the Freeze function for obtaining the ultrasound image through the input section 112 (step S130), then the control section 121 forms the ultrasound image based on the ultrasound echo signals acquired through the probe 110 and displays the formed image on the display 130 (step S 140).

Subsequently, if the 3D position sensor 111 detects the current position of the probe 110 (step S 150), then the control section 121 produces the position information corresponding to the detected current position (step S160). The control section 121 also checks whether the reference position information for detecting the position change is already set (step S170).

In step S 170, if it is determined that the reference position of the probe 110 is not set, then the control section 121 sets the reference position of the probe 110 based on the position information transmitted from the 3D position sensor 111 (step S180) and stores the information related to the set reference position (hereinafter referred to as the reference position information) into the memory 122 (step S 190).

On the other hand, if it is determined that the reference position is already set (step S 170), then the control section 121 compares the position information transmitted from the 3D position sensor 111 with the reference position information stored in the memory 122 and produces the gap between the position information and the reference position information (step S210). The control section 121 then checks whether the position information is the same as the reference position information, that is, whether the gap is 0 or not (step S220). If it is determined that the position information is the same as the reference position information, then the process goes back to step S150. Otherwise, the control section 121 changes, that is, the position of the ultrasound image displayed on the display 130 rotates based on the produced gap (step S230).

The control section 121 checks whether the ultrasound diagnostic system 100 is terminated. If it is determined that the ultrasound diagnostic system 100 is terminated, all the processes are terminated. Otherwise, the process goes back to step S 150.

(The second embodiment)

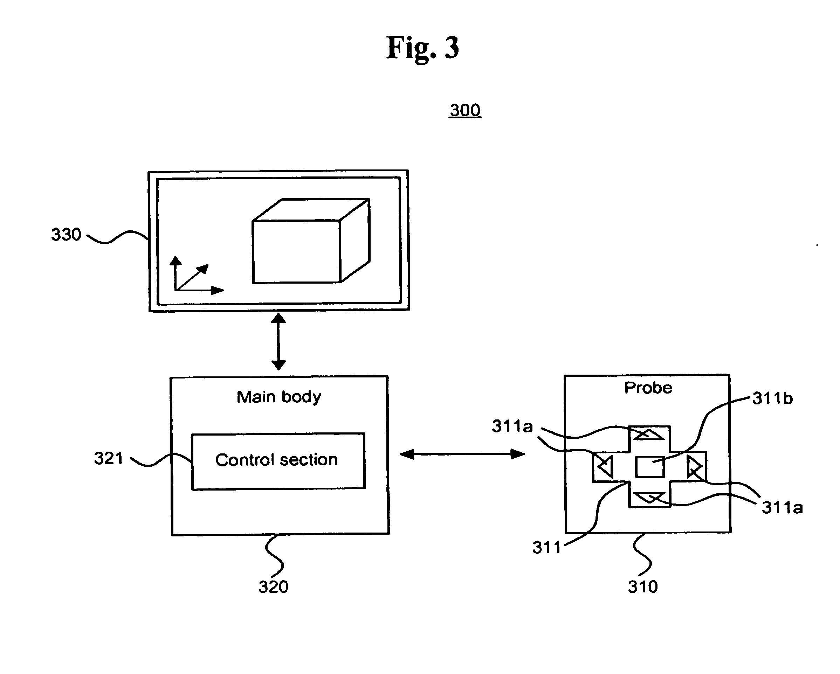

Fig. 3 is a block diagram for describing an ultrasound diagnostic system 300, which is constructed in accordance with the second embodiment of the present invention.

As shown in Fig. 3, the ultrasound diagnostic system 300 of the second embodiment comprises a probe 310, a main body 320 and a display 330. The probe 310 comprises a joystick 311. The probe 310 also comprises a plurality of 1D or 2D transducers (not shown), which can send a condensed ultrasound beam along the transmission scan line to a target (not shown) by properly adjusting the timing of applying input pulses to the transducers. Such transducers are also configured to receive the ultrasound echo signals reflected from the target at different timing.

The joystick 311 is used to rotate the ultrasound image, which is displayed on the display 330, in a plurality of directions (i.e., up, down, left and right). The joystick 311 comprises a button 311a for rotating the ultrasound image in up, down, left and right directions. It also comprises a selection button 311b for acquiring an ultrasound image and adjusting the acquired ultrasound image.

The main body 320 comprises a control section 321. The main body 320 also comprises a beam former, an image signal processor, a scan converter and an image processor (not shown).

The control section 321 controls the overall operation of the ultrasound diagnostic system 300. Particularly, the control section 321 is configured to perform the following: form an ultrasound image based on the ultrasound echo signals acquired from the probe 310; display the formed ultrasound image on the display 330; and change the position of (i.e., rotates) the ultrasound image displayed on the display 330 based on the input information of the joystick 311.

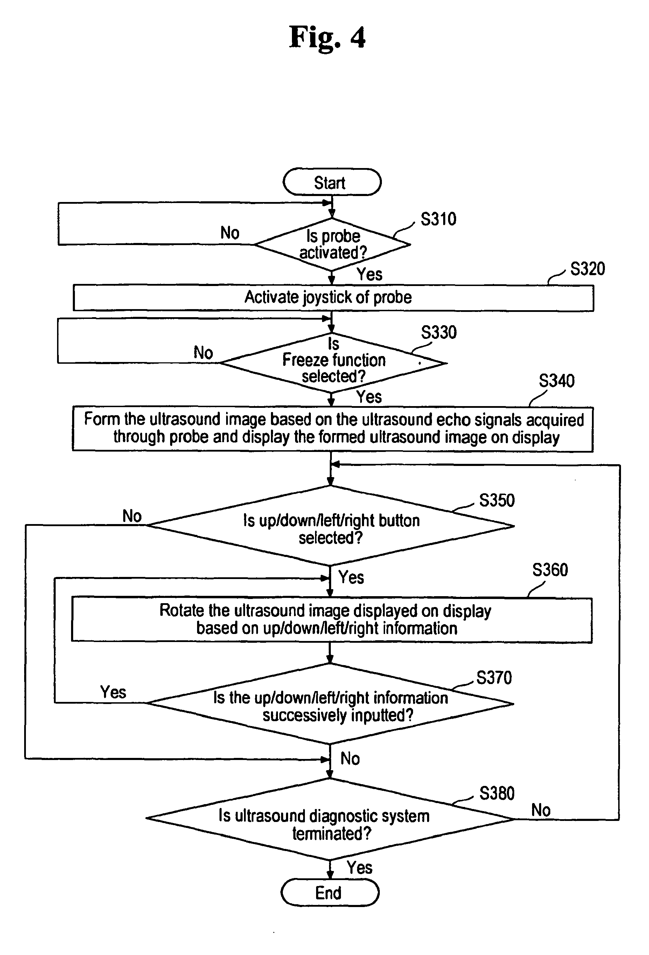

The operation of the ultrasound diagnostic system, which is in accordance with the second embodiment of the present invention, will be described below in view of Figs. 3 and 4. Fig. 4 is a flowchart for describing the operation of the ultrasound diagnostic system, which is in accordance with the second embodiment of the present invention.

As illustrated in Fig. 4, if a user activates the probe 310 by operating the ultrasound diagnostic system 300 (step S310), the control section 321 activates the joystick 311 of the probe 310 (step S320). If the user then selects the Freeze function for obtaining the ultrasound image through a selection button 311b of the joystick 311 (step S330), then the control section 321 forms the ultrasound image based on the ultrasound echo signals acquired through the probe 310 and displays the formed ultrasound image on the display 330 (step S340).

Subsequently, the control section 321 checks whether the user selects the directional button 311a of the joystick 311. If the user selects the directional button 311a of the joystick 311 in step S350, then the control section 321 rotates the ultrasound image, which is displayed on the display 330, based on the information corresponding to the selected direction button 311a (hereinafter referred to as the "up/down/left/right information") (step S360). However, if the directional button 311 a is not selected in step S350, then the process goes to step S380.

In step S370, the control section 321 checks whether the up/down/left/right information is successively inputted or not. If it is determined that the up/down/left/right information is successively inputted, step S360 is performed. If it is determined that the up/down/left/right information is not successively inputted, the process goes to step S380. In step S380, it is checked whether the ultrasound diagnostic system 300 is terminated or not. If it is determined that the ultrasound diagnostic system 300 is terminated, all the processes are terminated. If it is determined that the ultrasound diagnostic system 300 is not terminated, the process goes back to step S350.

(The third embodiment)

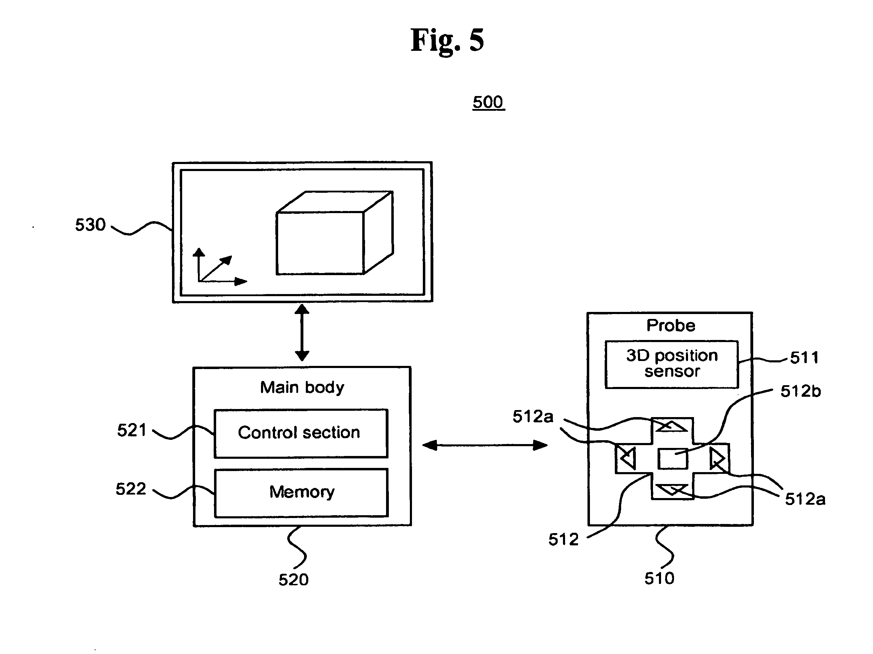

Fig. 5 is a block diagram for describing an ultrasound diagnostic system 500, which is constructed in accordance with the third embodiment of the present invention. As shown in Fig. 5, the ultrasound diagnostic system 500 of the third embodiment comprises a probe 510, a main body 520 and a display 530. The probe 510 comprises a 3D position sensor 511 and a joystick 512. The probe 510 also comprises a plurality of 1D or 2D transducers (not shown), which can send a condensed ultrasound beam along the transmission scan line to a target (not shown) by properly adjusting the timing of applying input pulses to the transducers. Such transducers are further configured to receive the ultrasound echo signals reflected from the target at different timing.

The 3D position sensor 511 detects the present position of the probe 510 and generates the position information of the probe 510. Any device may be used as the 3D position sensor 511 so long as the device can detect the position of the probe 510. For example, a gyro can be used as the 3D position sensor 511.

The joystick 512 is used to rotate the ultrasound image displayed on the display 530 in various directions (i.e., up, down, left and right). The joystick 512 comprises an up/down/left/right button 512a for rotating the ultrasound image in a number of directions, as well as a selection button 512b for acquiring an ultrasound image and adjusting the acquired ultrasound image.

The main body 520 comprises a control section 521 and a memory 522. The main body 520 is configured to perform the following: form the ultrasound image based on the ultrasound echo signals acquired from the probe 510; display the formed ultrasound image on the display 530; and rotate the ultrasound image displayed on the display 530 according to the change of position, that is, the rotation of the probe 510. The main body 520 also comprises a beam former, an image signal processor, a scan converter and an image processor (not shown).

The control section 521 controls the overall operation of the ultrasound diagnostic system 500. Particularly, the control section 521 rotates the ultrasound image displayed on the display 530 based on the position information transmitted from the 3D position sensor 511 of the probe 510 and the up/down/left/right information transmitted from the up/down/left/right button 512a of the joystick 512. The memory 522 stores the reference position information for determining the position change of the probe 510. The reference position information will be described below in detail.

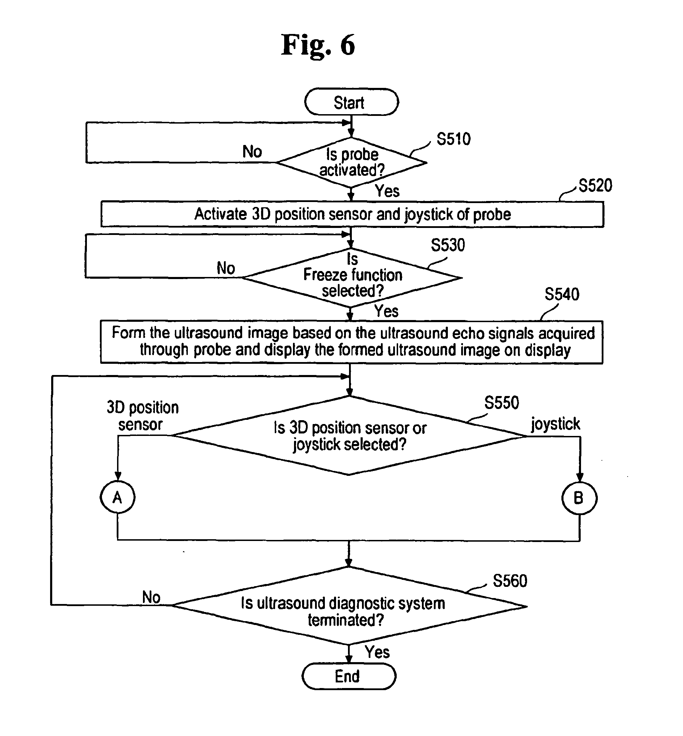

The operation of the ultrasound diagnostic system, which is in accordance with the second embodiment of the present invention, will be described below in view of Figs. 5 to 8. Fig. 6 is a flowchart for describing the operation of the ultrasound diagnostic system, which is constructed in accordance with the third embodiment of the present invention.

As illustrated in Fig. 6, if a user activates the probe 510 by operating the ultrasound diagnostic system 500 (step S510), then the control section 521 activates the 3D position sensor 511 and the joystick 512 of the probe 510 (step S520). If the user then selects the Freeze function for obtaining the ultrasound image through a selection button 512b of the joystick 512 (step S530), then the control section 521 forms the ultrasound image based on the ultrasound echo signals acquired through the probe 510 and displays the formed ultrasound image on the display 530 (step S540).

Subsequently, the control section 521 checks whether the user selects the 3D position sensor 511, that is, whether the user rotates the probe 510, or whether the user selects the joystick 512, that is, whether the user selects the buttons 512a and 512b of the joystick 512 (step S550).

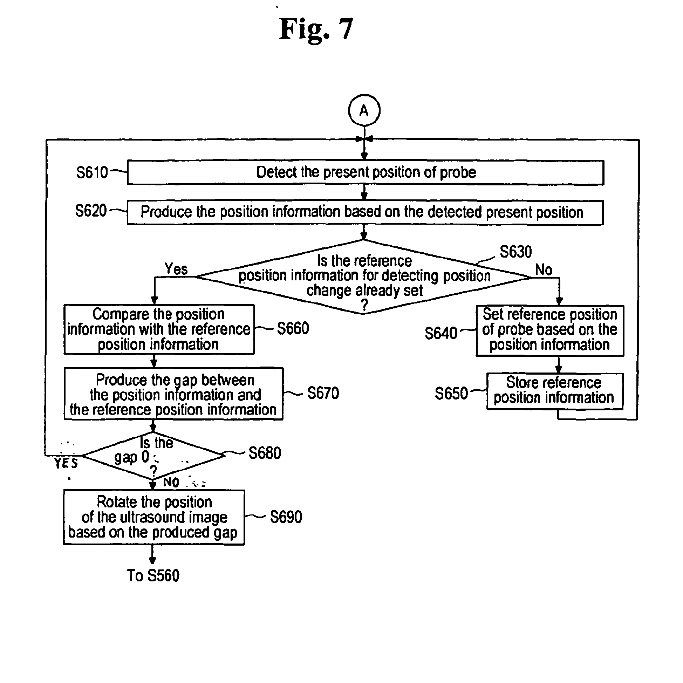

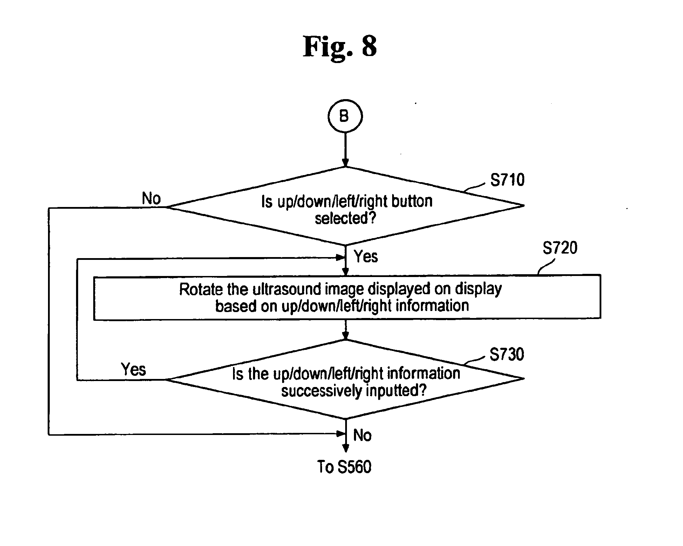

In step S550, if it is determined that the user selected the 3D position sensor 511, then process A is performed. If it is determined that the user selected the joystick 512, then process B is performed. Processes A and B will be described below in detail while referring to Figs. 7 and 8.

Subsequently, the control section 521 checks whether the ultrasound diagnostic system 500 is terminated or not (step S560). If it is determined that the ultrasound diagnostic system 500 is terminated, all the processes are terminated. If it is determined that the ultrasound diagnostic system 500 is not terminated, the process goes back to step S550.

Fig. 7 is a flowchart for describing the operation of rotating the ultrasound image by using the 3D position sensor according to the third embodiment of the present invention. As illustrated in Figs. 6 and 7, if the user selects the 3D position sensor 511, the control section 521 enables the 3D position sensor 511 to detect the present position of the probe 510 (step S610) and produce the position information corresponding to the detected present position (step S620). Subsequently, the control section 521 checks whether the reference position information for sensing the change of the position of the probe 510 is already set or not (step S630).

If it is determined that the reference position is not set, then the control section 521 sets the reference position of the probe 510 based on the position information transmitted from the 3D position sensor 511 (step S640) and stores the set reference position information into the memory 522 (step S650).

On the other hand, if it is determined that the reference position is set, then the control section 521 compares the position information transmitted from the 3D position sensor 511 with the reference position information stored in the memory 522 (step S660). It then produces the gap between the position information and the reference position information (step S670). The control section 121 then checks whether the position information is the same as the reference position information, that is, whether the gap is 0 or not (step S680). If it is determined that the position information is the same as the reference position information, then the process goes back to step S610. Otherwise, the control section 521 changes, that is, the position of the ultrasound image displayed on the display 530 is rotated based on the produced gap (step S690).

Fig. 8 is a flowchart for describing the operation of rotating the ultrasound image by using the joystick according to the third embodiment of the present invention. As illustrated in Fig. 8, if the user selects the joystick 512, the control section 521 checks whether the up/down/left/right button 512a of the joystick 512 is selected or not (step S710).

If it is determined that the up/down/left/right button 512a is not selected, then the process goes back to step S560. Otherwise, the control section 521 rotates the ultrasound image displayed on the display 530 based on the up/down/left/right information corresponding to the selected up/down/left/right button 512a (step S720).

In step S730, the control section 5021 checks whether the up/down/left/right information is successively inputted or not. If it is determined that the up/down/left/right information is successively inputted, step S720 is performed. If it is determined that the up/down/left/right information is not successively inputted, the process goes to step S560.

While the present invention has been described and illustrated with respect to a preferred embodiment of the invention, it will be apparent to those skilled in the art that variations and modifications are possible without deviating from the broad principles and teachings of the present invention which should be limited solely by the scope of the claims appended hereto.

## Claims

1. An ultrasound diagnostic system (100, 300, 500), comprising: a probe (110, 310, 510); input means (111, 112, 311, 512) for receiving information for use in rotating an ultrasound image in a predetermined direction; a main body (120, 320, 520) comprising a beam former, an image signal processor, a scan converter a memory (122) and an image processor, for forming the ultrasound image based on ultrasound echo signals acquired through the probe (110, 310, 510); a display (130, 330, 530) for displaying the ultrasound image; and a control section (121, 321, 521) for rotating the ultrasound image on the display (130, 330, 530) based on the information from the input means (111, 112, 311, 512),
**characterized in that** the input means (111, 112, 311, 512) is attached to the probe (110, 310, 510), and the control section (121, 321, 521) is included in the main body (120, 320, 520).

2. The ultrasound diagnostic system (100) as recited in claim 1, wherein the input means (112, 311, 512) comprises:
a joystick (311, 512) for receiving up/down/left/right information to rotate the ultrasound image in up/down/left/right directions.

3. The ultrasound diagnostic system (100) as recited in claim 1, wherein the input means (112, 311, 512) comprises:
a position sensor (111, 511) for generating position information of the probe (110, 310, 510) by detecting position of the probe (110, 310, 510).

4. The ultrasound diagnostic system (100) as recited in claim 1, wherein the input means (112, 311, 512) comprises:
a joystick (512) for receiving up/down/left/right information to rotate the ultrasound image in up/down/left/right direction; and
a position sensor (511) for generating position information of the probe (110, 310, 510) by detecting position of the probe (110, 310, 510).

5. The ultrasound diagnostic system (100) as recited in claim 3 or 4, wherein the position sensor comprises:
a reference position information generation section for determining a reference position of the probe (110, 310, 510) and generating reference position information corresponding to the determined reference position; and
a position information generation section for generating position information of the probe (110, 310, 510) based on the reference position information.

6. The ultrasound diagnostic system (100) as recited in claim 5, further comprising storage means for storing the reference position information.

7. A method for rotating an ultrasound image by using an ultrasound diagnostic system (100, 300, 500) according to claim 1, , comprising the steps of:
a) activating the probe (110, 310, 510) and the input means (112, 311, 512);
b) forming the ultrasound image based on ultrasound echo signals acquired through the probe (110, 310, 510) by using the main body (120), 320, 520) and displaying the ultrasound image on the display (130, 330, 530);
c) receiving information for rotating the displayed ultrasound image through the input means (112, 311, 512); and
d) rotating the ultrasound image based on the information by using the control section (121, 321, 521).

8. The method as recited in claim 7, wherein the step c) comprises the step of: receiving information for rotating the ultrasound image in up/down/left/right directions.

9. The method as recited in claim 7, wherein the step c) comprises the steps of:
c1) generating reference position information by determining a reference position of the probe (110, 310, 510);
c2) generating present position information by detecting a present position of the probe (110, 310, 510); and
c3) generating rotation information for rotating the ultrasound image based on the reference position information and the present position information.

10. The method as recited in claim 7, wherein the step e) comprises the steps of:
e1) analyzing information inputted through the input means (112, 311, 512);
e2) if the information is information for rotating the ultrasound image in up/down/left/right directions, performing the step d);
e3) if the information is position information of the probe (110, 310, 510), generating reference position information by determining a reference position of the probe (110, 310, 510);
e4) generating present position information by detecting a present position of the probe (110, 310, 510); and
e5) generating rotation information for rotating the ultrasound image based on the reference position information and the present position information.

## Patentansprüche

1. Ultraschalldiagnosesystem (100,300,500), welches Folgendes aufweist:
eine Messsonde (110,310,510);
eine Eingabeeinrichtung (111,112,311,512) zum Empfangen von Informationen zur Verwendung bei der Rotation eines Ultraschallbilds in einer vorbestimmten Richtung;
einen Grundkörper (120,320,520), der einen Strahlformer, einen Bildsignalprozessor, einen Scankonverter, einen Speicher (122) und einen Bildprozessor zum Bilden des Ultraschallbilds basierend auf durch die Messsonde (110,310,510) erhaltenen Ultraschallechosignalen aufweist;
eine Anzeige (130,330,530) zum Anzeigen des Ultraschallbilds;
und einen Steuerungsabschnitt (121,321,521) zum Rotieren des Ultraschallbilds auf der Anzeige (130,330,530) basierend auf den Informationen von der Eingabeeinrichtung (111,112,311,512),
**dadurch gekennzeichnet, dass**
die Eingabeeinrichtung (111,112,311,512) an der Messsonde (110,310,510) angebracht ist und der Steuerungsabschnitt (121,321,521) in dem Grundkörper (120,320,520) enthalten ist.

2. Ultraschalldiagnosesystem (100) nach Anspruch 1, wobei die Eingabeeinrichtung (112,311,512) Folgendes aufweist:
einen Joystick (311,512) zum Empfangen von Aufwärts-/Abwärts-/Links-/Rechts-Informationen, um das Ultraschallbild in einer Richtung nach oben/nach unten/nach links/nach rechts zu rotieren.

3. Ultraschalldiagnosesystem (100) nach Anspruch 1, wobei die Eingabeeinrichtung (112,311,512) Folgendes aufweist:
einen Positionssensor (111,511) zum Erzeugen von Positionsinformationen der Messsonde (110,310,510) durch Detektieren der Position der Messsonde (110, 310,510).

4. Ultraschalldiagnosesystem (100) nach Anspruch 1, wobei die Eingabeeinrichtung (112,311,512) Folgendes aufweist:
einen Joystick (512) zum Empfangen von Aufwärts-/Abwärts-/Links-/Rechts-Informationen, um das Ultraschallbild in einer Richtung nach oben/nach unten/nach links/nach rechts zu rotieren; und
einen Positionssensor (511) zum Erzeugen von Positionsinformationen der Messsonde (110,310,510) durch Detektieren der Position der Messsonde (110, 310,510).

5. Ultraschalldiagnosesystem (100) nach Anspruch 3 oder 4, wobei der Positionssensor Folgendes aufweist:
einen Referenzpositionsinformationserzeugungsabschnitt zum Ermitteln einer Referenzposition der Messsonde (110,310,510) und Erzeugen von Referenzpositionsinformationen, welche der ermittelten Referenzposition entsprechen; und
einen Positionsinformationserzeugungsabschnitt zum Erzeugen von Positionsinformationen der Messsonde (110,310,510) basierend auf den Referenzpositionsinformationen.

6. Ultraschalldiagnosesystem (100) nach Anspruch 5, welches des Weiteren eine Speichereinrichtung zum Speichern der Referenzpositionsinformationen aufweist.

7. Verfahren zum Rotieren eines Ultraschallbilds unter Verwendung eines Ultraschalldiagnosesystems (100,300,500) nach Anspruch 1, welches die folgenden Schritte aufweist:
a) Aktivieren der Messsonde (110,310,510) und der Eingabeeinrichtung (112,311,512);
b) Bilden des Ultraschallbilds basierend auf durch die Messsonde (110, 310,510) unter Verwendung des Grundkörpers (120,320,520) erhaltenen Ultraschallechosignalen und Anzeigen der Ultraschallsignale auf der Anzeige (130,330,530);
c) Empfangen von Informationen zum Rotieren des angezeigten Ultraschallbilds durch die Eingabeeinrichtung (112,311,512); und
d) Rotieren des Ultraschallbilds basierend auf den Informationen unter Verwendung des Steuerungsabschnitts (121,321,521).

8. Verfahren nach Anspruch 7, wobei der Schritt c) den folgenden Schritt aufweist:
Empfangen von Informationen zum Rotieren des Ultraschallbilds in einer Richtung nach oben/nach unten/nach links/nach rechts.

9. Verfahren nach Anspruch 7, wobei der Schritt c) die folgenden Schritte aufweist:
c1) Erzeugen von Referenzpositionsinformationen durch Ermitteln einer Referenzposition der Messsonde (110,310,510);
c2) Erzeugen von aktuellen Positionsinformationen durch Ermitteln einer aktuellen Position der Messsonde (110,310,510); und
c3) Erzeugen von Rotationsinformationen zum Rotieren des Ultraschallbilds basierend auf den Referenzpositionsinformationen und den derzeitigen Positionsinformationen.

10. Verfahren nach Anspruch 7, wobei der Schritt e) die folgenden Schritte aufweist:
e1) Analysieren von durch die Eingabeeinrichtung (112,311,512) eingegebenen Informationen;
e2) falls die Informationen Informationen zum Rotieren des Ultraschallbilds in eine Richtung nach oben/nach unten/nach links/ nach rechts sind, Durchführen des Schritts d);
e3) falls die Informationen Positionsinformationen der Messsonde (110, 310,510) sind, Erzeugen von Referenzpositionsinformationen durch Ermitteln einer Referenzposition der Messsonde (110,310,510);
e4) Erzeugen von aktuellen Positionsinformationen durch Ermitteln einer aktuellen Position der Messsonde (110,310,510); und
e5) Erzeugen von Rotationsinformationen zum Rotieren des Ultraschallbilds basierend auf den Referenzpositionsinformationen und den derzeitigen Positionsinformationen.

## Revendications

1. Système de diagnostic à ultrasons (100, 300, 500), comprenant une sonde (110, 310, 510), des moyens d'entrée (111, 112, 311, 512) pour recevoir une information à utiliser en tournant une image ultrasonique dans une direction prédéterminée, un corps principal (120, 320, 520) comprenant un formateur de faisceaux, un processeur de signaux image, un convertisseur de balayage, une mémoire (122), et un processeur d'image, pour former l'image ultrasonique sur la base de signaux d'échos ultrasoniques acquis par l'intermédiaire de la sonde (110, 310, 510), un écran (130, 330, 530) pour afficher l'image ultrasonique, et une section de commande (121, 321, 521) pour faire tourner l'image ultrasonique sur l'écran (130, 330, 530) sur la base de l'information issue des moyens d'entrée (111, 112, 311, 512), **caractérisé en ce que** les moyens d'entrée (111, 112, 311, 512) sont attachés à la sonde (110, 310, 510), et la section de commande (121, 321, 521) est incluse dans le corps principal (120, 320, 520).

2. Système de diagnostic à ultrasons (100) tel qu'énoncé dans la revendication 1, dans lequel les moyens d'entrée (112, 311, 512) comprennent:
une manette (311, 512) pour recevoir une information haut/bas/gauche/droite pour faire tourner l'image ultrasonique dans les directions haut/bas/gauche/droite./

3. Système de diagnostic à ultrasons (100) tel qu'énoncé dans la revendication 1, dans lequel les moyens d'entrée (112, 311, 512) comprennent:
un détecteur de position (111, 511) pour générer une information de position de la sonde (110, 310, 510) par détection de la position de la sonde (110, 310, 510).

4. Système de diagnostic à ultrasons (100) tel qu'énoncé dans la revendication 1, dans lequel les moyens d'entrée (112, 311, 512) comprennent:
une manette (512) pour recevoir une information haut/bas/gauche/droite pour faire tourner l'image ultrasonique dans la direction haut/bas/gauche/droite, et
un détecteur de position (511) pour générer une information de position de la sonde (110, 310, 510) par détection de la position de la sonde (110, 310, 510).

5. Système de diagnostic à ultrasons (100) tel qu'énoncé dans la revendication 3 ou 4, dans lequel le détecteur de position comprend : a une section de génération d'information de position de référence pour déterminer une position de référence de la sonde (110, 310, 510) et générer une information de position de référence correspondant à la position de référence déterminée, et
une section de génération d'information de position pour générer une information de position de la sonde (110, 310, 510) sur la base de l'information de position de référence.

6. Système de diagnostic à ultrasons (100) tel qu'énoncé dans la revendication 5, comprenant en outre des moyens de stockage pour stocker l'information de position de référence.

7. Procédé pour faire tourner une image ultrasonique en utilisent un système de diagnostic à ultrasons (100, 300, 500) selon la revendication 1, comprenant les étapes de:
a) activation de la sonde (110, 310, 510) et des moyens d'entrée (112, 311, 512),
b) formation de l'image ultrasonique sur la base de signaux d'échos ultrasoniques acquis par l'intermédiaire de la sonde (110, 310, 510) en utilisant le corps principal (120, 320, 520) et affichage de l'image ultrasonique sur l'écran (130, 330, 530),
c) réception d'une information pour faire tourner l'image ultrasonique affichée par l'intermédiaire des moyens d'entrée (112, 311, 512), et
d) rotation de l'image ultrasonique sur la base de l'information en utilisant la section de commande (121, 321, 521).

8. Procédé tel qu'énoncé dans la revendication 7, dans lequel l'étape c) comprend l'étape de réception d'une information pour faire tourner l'image ultrasonique dans les directions haut/bas/gauche/droite.

9. Procédé tel qu'énoncé dans la revendication 7, dans lequel l'étape c) comprend les étapes de:
c1) génération d'une information de position de référence par détermination d'une position de référence de la sonde (110, 310, 510),
c2) génération d'une information de position présente par détection d'une position présente de la sonde (110, 310, 510), et
c3) génération d'une information de rotation pour faire tourner l'image ultrasonique sur la base de l'information de position de référence et de l'information de la position présente.

10. Procédé tel qu'énoncé dans la revendication 7, dans lequel l'étape e) comprend les étapes de:
e1) analyse d'une information entrée par l'intermédiaire des moyens d'entrée (112, 311, 512),
e2) si l'information est une information pour faire tourner l'image ultrasonique dans les directions haut/bas/gauche/droite, exécution de l'étape d),
e3) si l'information est une information de position de la sonde (110, 310, 510), génération d'une information de position de référence par détermination d'une position de référence de la sonde (110, 310, 510),
e4) génération d'une information de position présente par détection d'une position présente de la sonde (110, 310, 510), et
e5) génération d'une information pour faire tourner l'image ultrasonique sur la base de l'information de position de référence et de l'information de position présente.
